# EUROPÄISCHE PATENTSCHRIFT

(11) **EP 3 227 011 B1**
(45) Veröffentlichungstag und Bekanntmachung des Hinweises auf die Patenterteilung: **03.04.2019**
(21) Anmeldenummer: 15781947.5
(22) Anmeldetag: 20.10.2015
(51) Int. Cl.: B01F 7/18, B01F 15/00, C12M 1/02

(54) **MISCHVORRICHTUNG MIT STRÖMUNGSBRECHER**
MIXING DEVICE HAVING A FLOW BREAKER
DISPOSITIF DE MÉLANGE AVEC BRISE-FLUX

(30) Priorität: 02.12.2014 DE 102014117658
(43) Veröffentlichungstag der Anmeldung: 11.10.2017
(73) Patentinhaber: Sartorius Stedim Biotech GmbH, 37079 Göttingen (DE)
(72) Erfinder: ZAHNOW, Christian, 37085 Goettingen (DE); RUHL, Sebastian, 36043 Fulda (DE); GRELLER, Gerhard, 37085 Goettingen (DE)
(74) Vertreter: Prinz & Partner mbB
(86) Internationale Anmeldenummer: PCT/EP2015/074263
(87) Internationale Veröffentlichungsnummer: WO 2016/087113

(56) Entgegenhaltungen:
- WO-A1-2014/003640
- WO-A2-2007/134267

## Beschreibung

Die Erfindung betrifft eine Mischvorrichtung mit einem flexiblen Behälter und wenigstens einem Rührorgan sowie wenigstens einem Strömungsbrecher.

Mischvorrichtungen finden in verschiedenen technischen Gebieten Anwendung. Ohne die Erfindung einzuschränken, wird im Folgenden auf die Anwendung von Mischvorrichtungen als Bioreaktoren eingegangen, in denen bestimmte Mikroorganismen oder pflanzliche oder tierische Zellen unter möglichst optimalen Bedingungen kultiviert werden sollen.

Ein häufig anzutreffender Bioreaktortyp ist der Rührkessel mit einem starren Behälter, der meistens aus Edelstahl, seltener aus Glas oder glasfaserverstärktem Kunststoff gebildet ist. Ein Rührwerk mit einem oder mehreren im Behälter angeordneten Rührorganen sorgt für eine Durchmischung der im Behälter befindlichen Flüssigkeit. Bei der Zugabe von Substanzen zu der im Behälter befindlichen Flüssigkeit, wie etwa einem Korrekturmittel, ist eine schnelle, gleichmäßige Verteilung wünschenswert. Gleiches gilt im Falle einer Begasung der Flüssigkeit; die eingebrachten Gasblasen sollen zudem möglichst lange in der Flüssigkeit gehalten werden.

Um während des Rührens die beim Mitrotieren der im Behälter befindlichen Flüssigkeit auftretende Bildung von Tromben zu verhindern, und um eine höhere Turbulenz zu erzeugen, können Hindernisse in Form von Schikanen, sogenannte Strömungsbrecher (engl. baffles) an der Behälterwand installiert werden. Diese in den Behälter hineinragenden Bauteile "stören" den gleichmäßigen Strömungsverlauf der Flüssigkeit im Behälter und können dadurch die Durchmischung erheblich verbessern. Im Hinblick auf die Kultivierung von Mikroorganismen in Bioreaktoren ermöglichen die Strömungsbrecher deutlich höhere Rührgeschwindigkeiten. Dies wiederum erlaubt einen erhöhten Sauerstoffeintrag in die Flüssigkeit, welcher unerlässlich für diese Art der Kultivierung ist.

Ein Beispiel für einen Rührkessel mit Strömungsbrechern, die von der starren Behälterwand radial vorragen, ist in der US 7 547 135 B2 zu finden.

Um den Sterilisationsaufwand vor einem Einsatz eines Bioreaktors vor Ort zu vermeiden, kommen vermehrt vorsterilisierte Einwegbehälter mit flexiblen Wänden zum Einsatz. Diese beutelartigen Behälter lassen sich für den Transport platzsparend zusammenfalten und werden in der Regel vor dem Gebrauch in einen Stützbehälter eingesetzt, obwohl auch andere Stützkonstruktionen zum Halten der teilweise mit sehr großen Flüssigkeitsmengen befüllten Einwegbehälter bekannt sind. Vorsterilisierte Einwegbehälter können bereits mit einer Mehrzahl an vormontierten Schläuchen ausgestattet sein. Außerdem können vorsterilisierte Rührorgane in die Beutel integriert sein, die z. B. über Magnetkörper im Beutelinneren mit einem externen Rührmotor des Bioreaktors gekoppelt sind.

Aus der WO 2005/118771 A2 und der WO 2007/124847 A1 sind Behälteranordnungen der vorgenannten Art bekannt. Zur Verbesserung des Mischvorgangs sind auch hier Strömungsbrecher vorgesehen, die an der Innenwand des Stützbehälters angebracht sind. Die seitliche Wandung des flexiblen Einwegbehälters schmiegt sich an die Innenwand des Stützbehälters mit den angeformten Strömungsbrechern an, sodass die Strömungsbrecher - von der Wandung des Einwegbehälters überdeckt - ihre Funktion erfüllen können.

Diese Lösung hat zwar den Vorteil, dass der Einwegbehälter einfach gestaltet werden kann, da er selbst nicht mit Strömungsbrechern versehen werden muss, und dass die Strömungsbrecher der Stützbehälter immer wieder verwendet werden können. Die Anzahl, Anordnung und Form der Strömungsbrecher am Stützbehälter sind jedoch fest vorgegeben und können nicht variiert werden. Außerdem ist zu beachten, dass die Wandung des Einwegbehälters an den Strömungsbrechern stark deformiert wird und deshalb einer großen Belastung ausgesetzt ist. Ein weiterer Nachteil dieser Konstruktion besteht darin, dass die Strömungsbrecher die effektive Auflagefläche der Einwegbehälterwandung auf der Innenwand des Stützbehälters verringert, was eine Wärmeabfuhr vom Einwegbehälter auf den Stützbehälter erschwert.

Ein ähnlicher Aufbau einer Behälteranordnung wie in den oben genannten Dokumenten WO 2005/118771 A2 und WO 2007/124847 A1 ist auch in der WO 2009/074213 A1 gezeigt. Hier können die Strömungsbrecher am Stützbehälter zusätzlich mit einem Temperierungssystem ausgestattet sein, um eine gleichmäßige und schnelle Temperierung zu fördern. Hierzu weisen die Strömungsbrecher parallele Rohre auf, durch die eine Flüssigkeit mit vorgegebener Temperatur geleitet werden kann.

Eine weitere Alternative für das Vorsehen von Strömungsbrechern bei der Verwendung eines Einwegbehälters wäre das Einschweißen eines starren Kunststoffteils in den flexiblen Beutel. Dies ist aber aufgrund des Transports des Einwegbehälters im komprimierten Zustand nicht oder zumindest nicht ohne Platzeinbuße und zusätzliche Sicherungsmaßnahmen möglich.

Die US 8 500 322 B2 schlägt eine Mischvorrichtung mit einem Kunststoffbeutel vor, der in einen starren Stützbehälter eingesetzt ist und einen Strömungsbrecher in Form einer perforierten Folienbahn aufweist. Die Folienbahn erstreckt sich zwischen wenigstens zwei inneren Oberflächen des Beutels, wie etwa von der oberen zur unteren Oberfläche oder zwischen zwei seitlichen Oberflächen. Die Folienbahn durchquert also immer den gesamten Beutel. Die Perforation erlaubt es der vom Rührwerk in Bewegung versetzten Flüssigkeit nur teilweise, durch die Folienbahn hindurch zu strömen.

Des Weiteren ist aus der WO 2014/172047 A1 ein Einweg-Bioreaktor mit einem flexiblen Strömungsbrecher bekannt, der zwei sich kreuzende Schenkel aufweist. Die Enden des Strömungsbrechers sind allesamt an der inneren Wand des Bioreaktors befestigt.

Die JP2014-121302A zeigt einen Zellkulturbehälter mit einem Rührwerk und einem Strömungsbrechern aus Kunststoff, der sich im Behälter in Richtung der Rührachse erstreckt. Der Strömungsbrecher ist zwischen gegenüberliegende vertikal verlaufende Ränder zweier Filmabschnitte eingefügt, aus denen der Hauptbehälterabschnitt gebildet ist. Die Befestigung des Strömungsbrechers an den Filmrändern erfolgt durch Verschweißen (Wärmeversiegelung). Die Stabilität dieser Befestigung ist jedoch begrenzt. Die Strömungsbrecher sind mit Löchern versehen, durch die die zu kultivierende Substanz passieren kann. Die Löcher können sich wegen der dadurch erzeugten Scherkräfte als ungünstig für biologisches Material erweisen. Die Verwendung von perforierten Strömungsbrechern dürfte bevorzugt bei Medien mit Mikrocarrier-Zellkulturen zu sehen sein.

Die WO 2007/134267 A2 und die WO 2014/003640 A1 offenbaren jeweils eine Mischvorrichtung mit den Merkmalen des Oberbegriffs des Anspruchs 1. Bei beiden Vorrichtungen sind auf der Innenseite an einer umlaufenden Seitenwand eines flexiblen Behälters Strömungsbrecher gebildet, die ein in das Innere des Behälters ragendes freies Ende aufweisen.

Aufgabe der Erfindung ist es, bei einer Mischvorrichtung mit einem flexiblen Behälter eine materialschonende, variabel vorgebbare Anordnung von Strömungsbrechern für eine optimale Durchmischung zu ermöglichen.

Gelöst wird diese Aufgabe durch eine Mischvorrichtung mit den Merkmalen des Anspruchs 1. Vorteilhafte und zweckmäßige Ausgestaltungen der erfindungsgemäßen Mischvorrichtung sind in den Unteransprüchen angegeben.

Die erfindungsgemäße Mischvorrichtung umfasst einen flexiblen Behälter, wenigstens ein Rührorgan und wenigstens einen Strömungsbrecher. Der Strömungsbrecher ist auf der Innenseite an einer umlaufenden Seitenwand des flexiblen Behälters gebildet (angebracht oder angeformt) und weist ein in das Innere des Behälters ragendes freies Ende auf.

Die Erfindung beruht auf der Erkenntnis, dass ein Strömungsbrecher, der an der Innenseite des flexiblen Behälters gebildet ist und frei in den Behälter ragt (sich also nicht wie bei der US 8 500 322 B2 vollständig bis zu einer gegenüberliegenden Seite des Behälters erstreckt), durch geeignete Maßnahmen so stabilisiert werden kann, dass er die gewünschte Funktion, nämlich eine gezielte Verbesserung der Durchmischung, in der gleichen Weise wie ein fest an einem starren Behälter angebrachter Strömungsbrecher ausüben kann. Es ist lediglich darauf zu achten, dass die Befestigungsstellen des erfindungsgemäßen Strömungsbrechers stabil genug sind, um ein Ausreißen auszuschließen. Bei geeigneter Gestaltung des Strömungsbrechers lassen sich solche beständigen Verbindungen durch Schweißen oder Kleben aber ohne größere Probleme realisieren.

Das der Erfindung zugrundeliegende Konzept ermöglicht die größtmögliche Flexibilität hinsichtlich der Anzahl, Anordnung und Form der Strömungsbrecher, da sie je nach Anforderung individuell gestaltet und angebracht werden können. Solche Anpassungsmöglichkeiten bieten fest installierte Strömungsbrecher nicht.

Die Strömungsbrecher der erfindungsgemäßen Mischvorrichtung können gegebenenfalls separat zur Verfügung gestellt werden, sodass erst am Einsatzort entschieden werden kann, welche und wie viele Strömungsbrecher wo am flexiblen Behälter der Mischvorrichtung angebracht werden. Im Falle von vorsterilisierten flexiblen Einwegbehältern werden diese vorkonfektioniert angeboten, d. h. die Strömungsbrecher sind bereits angebracht, bevor die flexiblen Behälter versandt werden. Der Vorteil hierbei ist, dass sich der flexible Behälter selbst mit den angebrachten Strömungsbrechern noch sehr gut zu einem kompakten Paket zusammenlegen lässt.

Ein weiterer wesentlicher Vorteil der Erfindung, insbesondere gegenüber Lösungen mit Beuteln, die in Stützbehälter mit daran angeformten Strömungsbrechern eingesetzt werden, besteht darin, dass der flexible Behälter keinen übermäßigen mechanischen Belastungen ausgesetzt ist. Da der flexible Behälter der erfindungsgemäßen Mischvorrichtung selbst die Strömungsbrecher trägt, muss er nicht durch externe Strömungsbrecher des Stützbehälters deformiert werden. Der flexible Behälter kann sich vielmehr gleichmäßig an die Innenwände des Stützbehälters anlegen, was in einem besseren Wärmeübertrag zwischen dem flexiblen Behälter und dem Stützbehälter resultiert.

Gemäß einer bevorzugten Ausführungsform der erfindungsgemäßen Mischvorrichtung weist der Strömungsbrecher vorzugsweise einen Mantel auf, der aus einem flexiblen Folienmaterial gebildet ist. Die Biegsamkeit des Mantels begünstigt das Zusammenlegen für den Transport. Für den Gebrauch als Strömungsbrecher werden in den Mantel entweder starre Elemente eingesetzt, und/oder es wird ein Fluid (Flüssigkeit oder Gas) eingebracht, das zur Formstabilisierung des Strömungsbrechers zumindest beiträgt. Auf beide Möglichkeiten wird später noch genauer eingegangen. Der flexible Mantel lässt sich somit in eine gewünschte Form bringen bzw. in einer gewünschten Form stabilisieren.

Der Mantel des Strömungsbrechers weist vorzugsweise einen doppellagigen Abschnitt auf. In einem solchen Abschnitt lassen sich leicht Hohlräume bzw. Fächer zur Aufnahme von starren Elementen oder eines Fluids bilden.

Gemäß der bevorzugten Ausgestaltung des Strömungsbrechers ist der Mantel, insbesondere dessen doppellagiger Abschnitt, durch eine vertikale Abtrennung in zwei Aufnahmeabschnitte unterteilt, wobei die vertikale Abtrennung als freies Ende des Strömungsbrechers in das Innere des Behälters ragt. (Die Bezeichnung "vertikal" bezieht sich auf die Gebrauchsstellung der Mischvorrichtung.) Der Mantel bildet in dieser Ausgestaltung - geometrisch vereinfacht ausgedrückt - die Seitenflächen eines geraden Prismas mit einem Dreieck als Grundfläche. Diese grundlegende Form des Strömungsbrechers hat sich bewährt und kann durch Veränderung der Seitenlängen und Winkel an die jeweiligen Bedürfnisse angepasst werden.

Wie bereits erwähnt können starre Elemente in den Mantel eingefügt werden, um die erforderliche Stabilität des Strömungsbrechers zu gewährleisten. Es bietet sich an, wenigstens eine formstabilisierende Platte im Mantel anzuordnen. Material, Form und Stärke der Platte können abhängig von den jeweiligen Anforderungen und den Abmessungen des Mantels passend gewählt werden.

Gemäß einer Weiterbildung der Erfindung weist der Mantel mehrere abgetrennte Fächer auf, in denen jeweils eine Platte aufgenommen ist. Diese Gestaltung basiert auf dem Vorteil, dass mehrere kleine Platten leichter zu handhaben sind als eine große. Außerdem ist bei der Verwendung mehrerer kleiner Platten ein kompakteres Zusammenlegen des Strömungsbrechers möglich. "Abgetrennte Fächer" soll hier bedeuten, dass eine Platte nicht eigenmächtig aus ihrem Fach herausrutschen kann, sodass die Position der jeweiligen Platte während des Betriebs festgelegt ist.

Zusätzlich oder alternativ zu den Platten kann der Mantel oder der gesamte Strömungsbrecher durch Befüllen mit einem Fluid in die gewünschte Form gebracht und/oder weiter stabilisiert werden. Hierzu ist vorgesehen, dass der Mantel bzw. der gesamte Strömungsbrecher gasdicht abgeschlossen ist und wenigstens einen Fluidanschluss aufweist.

Eine besonders vorteilhafte Ausführungsvariante ergibt sich, wenn der Mantel bzw. der gesamte Strömungsbrecher gasdicht abgeschlossen ist und zwei Fluidanschlüsse aufweist, die vorzugsweise an vertikal entgegengesetzten Enden des Mantels bzw. Strömungsbrechers angeordnet sind. Ein solcher Aufbau erlaubt es, den in das Behälterinnere ragenden Mantel bzw. den gesamten Strömungsbrecher kontinuierlich mit einem temperierten Fluid zu durchströmen. Auf diese Weise kann die Flüssigkeit im Behälterinneren auf eine gewünschte Temperatur gebracht und auf dieser Temperatur gehalten werden.

Die beiden Effekte "Formgebung/Stabilisierung durch Fluid" und "Temperierung durch Fluid" sind gut miteinander kombinierbar, im günstigsten Falle unter Verwendung derselben Fluidanschlüsse.

Zur Befestigung des Strömungsbrechers an der umlaufenen Seitenwand des flexiblen Behälters weist der Strömungsbrecher gemäß der Erfindung zwei Laschen auf. Diese können flächig mit der Seitenwand des flexiblen Behälters verbunden werden, insbesondere durch Schweißen oder Kleben, sodass bei der Einleitung der auf den Strömungsbrecher wirkenden Kräfte auf den flexiblen Behälter keine stabilitätsbedrohlichen Kraftspitzen auftreten.

Eine vorteilhafte Gestaltung sieht in diesem Zusammenhang vor, dass sich die Laschen auf entgegengesetzten Seiten eines dem Mantel gegenüberliegenden Grundabschnitts erstrecken. Dadurch wird eine im Idealfall symmetrische Befestigung auf beiden Seiten des Mantels erreicht.

Die Montage der Strömungsbrecher am flexiblen Behälter durch Schweißen oder Kleben ist am einfachsten, wenn gleiche Materialien miteinander verbunden werden. Deshalb sind der flexible Behälter und die Laschen vorzugsweise aus dem gleichen Material gebildet. Wenn auch die übrigen Bestandteile des Strömungsbrechers, insbesondere der Mantel (mit Ausnahme etwa darin aufgenommener starrer Elemente oder etwa vorhandener Schlauchanschlüsse und - leitungen), aus dem gleichen Material gebildet sind, vereinfacht sich die Herstellung des Strömungsbrechers weiter.

Für bestimmte Anwendungen ist es vorteilhaft, dass die im Behälter befindliche Flüssigkeit zwischen der Innenseite des flexiblen Behälters und dem Strömungsbrecher durchströmen kann. Dies kann durch entsprechend angeordnete Abstandshalter erreicht werden. Eine von der Innenwand beabstandete Anordnung des Strömungsbrechers kann im Hinblick auf eine noch bessere Durchmischung für zusätzliche Verwirbelungen und Turbulenzen sorgen.

Dieses Verhalten kann durch die Größe und den Zwischenraum zwischen vertikal und/oder horizontal voneinander beabstandeten Abstandshaltern weiter beeinflusst werden.

Der erfindungsgemäß vorgesehene Strömungsbrecher weist vorzugsweise keine Durchgangsöffnungen auf, sodass keine für biologisches Material ungünstige Scherkräfte entstehen können.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 eine seitliche Schnittansicht einer erfindungsgemäßen Mischvorrichtung mit Strömungsbrechern;
- Figur 2 eine Draufsicht auf einen Ausschnitt einer erfindungsgemäßen Mischvorrichtung;
- Figur 3 eine perspektivische Ansicht eines Ausschnitts einer erfindungsgemäßen Mischvorrichtung; und
- Figur 4 eine seitliche Schnittansicht einer erfindungsgemäßen Mischvorrichtung mit einem Strömungsbrecher gemäß einer Ausführungsvariante mit Abstandshaltern.

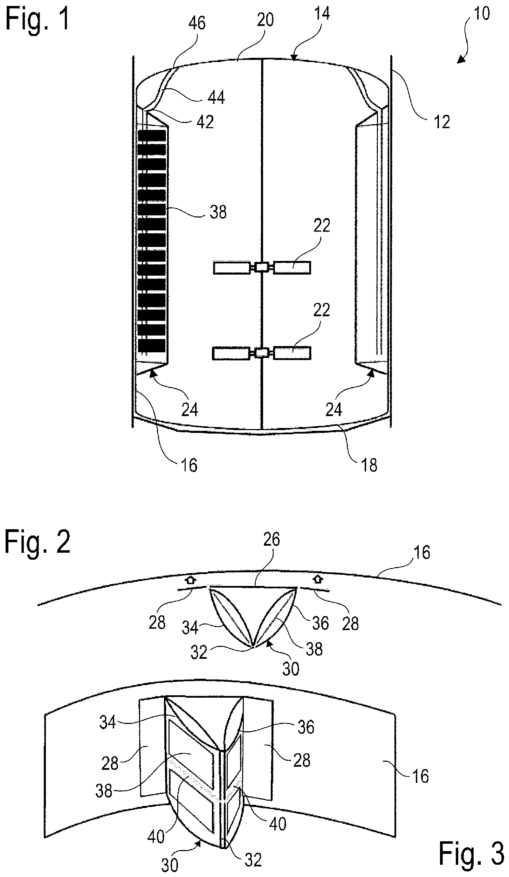

In Figur 1 ist eine Mischvorrichtung 10 dargestellt, die zur Verwendung als Bioreaktor vorgesehen ist. Die Mischvorrichtung umfasst einen im Wesentlichen zylindrischen, oben offenen Stützbehälter 12, in den ein Behälter 14 mit flexiblen Wänden 16, 18, 20 eingesetzt ist. Der flexible Behälter 14 ist dabei so angeordnet, dass seine Wände, insbesondere die umlaufende Seitenwand 16 und die Bodenwand 18, im Hinblick auf eine optimale Abstützung und einen optimalen Wärmeübertrag möglichst flächig an den gegenüberliegenden Innenwänden des Stützbehälters 12 anliegen.

Der flexible Behälter 14 ist hier ein Kulturgefäß in Form eines vorsterilisierten Einwegbeutels, der aus einer Folie gebildet ist. In den flexiblen Behälter 14 sind zwei Rührorgane 22 integriert, die Teile eines Rührwerks der Mischvorrichtung 10 sind. Selbstverständlich kann auch eine andere Anzahl von Rührorganen 22 vorgesehen sein.

An der umlaufenden Seitenwand 16 des flexiblen Behälters 14 sind innenseitig mehrere Strömungsbrecher 24 angebracht, die sich ins Behälterinnere erstrecken. Der Aufbau und die Anbringung der Strömungsbrecher 24 werden nachfolgend im Detail unter Bezugnahme auf die beispielhafte Darstellung eines Strömungsbrechers 24 in den Figuren 2 und 3 näher erläutert.

Der Strömungsbrecher 24 ist großteils aus einer Folie gebildet, die vorzugsweise aus demselben Material wie der flexible Behälter 14 besteht. Von einem Grundabschnitt 26 erstrecken sich auf entgegengesetzten Seiten zwei Laschen 28, mit denen der Strömungsbrecher 24 an der Seitenwand 16 des flexiblen Behälters 14 befestigt wird, insbesondere durch Schweißen.

Zwischen den Laschen erstreckt sich des Weiteren ein Mantel 30 in Form eines doppellagigen Folienabschnitts, der an die Laschen 28 angeschweißt oder auf sonstige Weise mit diesen verbunden oder einstückig mit diesen ausgeführt ist. Der doppellagige Abschnitt ist wiederum durch eine mittlere vertikale Abtrennung 32, beispielsweise eine Schweißnaht, in zwei Aufnahmeabschnitte 34, 36 unterteilt. In der Gebrauchsstellung der Mischvorrichtung 10 von oben betrachtet (siehe Figur 2) bilden die beiden Aufnahmeabschnitte 34, 36 und der Grundabschnitt 26 zusammen ein Dreieck, dessen Spitze, repräsentiert durch die vertikale Abtrennung 32, als freies Ende des Strömungsbrechers 24 in das Behälterinnere ragt.

In die Aufnahmeabschnitte 34, 36 sind starre Platten 38 eingesetzt. Die Aufnahmeabschnitte 34, 36 sind dafür durch mehrere im Wesentlichen horizontale Abtrennungen 40, beispielsweise Schweißnähte, so unterteilt, dass mehrere Fächer gebildet sind. In jedem dieser Fächer ist eine Platte 38 aufgenommen.

Die Strömungsbrecher 24 alleine lassen sich für den Transport flach ausbreiten oder platzsparend zusammenfalten, auch wenn die Platten 38 bereits eingesetzt sind. Die Montage der Strömungsbrecher 24 am flexiblen Behälter 14 kann dann am Einsatzort erfolgen, indem die vorgefertigten Strömungsbrecher 24 an den gewünschten Stellen am flexiblen Behälter 14 angebracht werden.

Bei vorsterilisierten Einwegbehältern ist hingegen vorgesehen, die Strömungsbrecher 24 bereits vor dem Versand der Mischvorrichtung 10 zu installieren, insbesondere wenn häufig eine bestimmte Konfiguration gewünscht wird, beispielsweise mit vier gleichen, gleichmäßig über den Innenumfang des flexiblen Behälters 14 verteilten Strömungsbrechern. In diesem Fall ist immer noch ein Zusammenlegen des flexiblen Behälters 14 zu einem verhältnismäßig kompakten Paket möglich.

Nachfolgend werden einige Ausführungsvarianten der Mischvorrichtung 10 in Bezug auf die Strömungsbrecher 24 beschrieben. Allgemein gilt, dass nicht zwingend alle Merkmale einer bestimmten Ausführungsvariante verwirklicht sein müssen. Andererseits können auch Merkmale verschiedener Ausführungsvarianten miteinander kombiniert werden.

Um einen Strömungsbrecher 24 in seine finale Form zu bringen, kann dieser zusätzlich oder alternativ zu den Platten 38 mit einer Flüssigkeit oder einem Gas (nachfolgend: Fluid) gefüllt werden. Hierzu ist, wie in Figur 1 angedeutet, der Strömungsbrecher 24 mit wenigstens einem Fluidanschluss 42 (Port) bestückt, welcher über eine Schlauchleitung 44 mit einem in der Deckenwand 20 des flexiblen Behälters 14 angeordneten Fluidanschluss 46 verbunden werden kann. Ansonsten ist der Strömungsbrecher 24 gasdicht abgeschlossen.

Soweit erforderlich erfolgt die Verteilung des zugeführten Fluids innerhalb der Strömungsbrecher 24 über weitere Schlauchleitungen oder sonstige Strömungsverbindungen zwischen den Fächern, in denen die Platten 38 aufgenommen sind. Genauso gut können aber auch separate Abschnitte in den Strömungsbrechern 24 oder die gesamten Strömungsbrecher 24 zur Aufnahme des Fluids vorgesehen sein. Wie bereits erwähnt kann im Falle einer Fluidstabilisierung auf die verstärkenden Platten 38 auch teilweise oder ganz verzichtet werden, wie in der rechten Hälfte von Figur 1 angedeutet.

Allgemein darf der Druck, der durch das Fluid eingebracht wird, die Stabilität der Strömungsbrecher 24, insbesondere der Abtrennungen 32, 40 (Schweißnähte) - soweit vorhanden - und der Befestigungsstellen, nicht gefährden. In der Praxis hängt der maximal zulässige Druck maßgeblich vom verwendeten Folienmaterial ab.

Zusätzlich oder alternativ kann das zugeführte Fluid zum Temperieren des flexiblen Behälters 14 bzw. der darin befindlichen Flüssigkeit genutzt werden. In diesem Fall ist ein zusätzlicher Fluidanschluss am Mantel 30 bzw. an einer anderen den Innenraum des Strömungsbegrenzers 24 abgrenzenden Wandung vorgesehen, durch den das Fluid an anderer Stelle aus dem Strömungsbrecher 24 wieder herausgeführt werden kann, sodass ein kontinuierliches Durchströmen des Strömungsbrechers 24 mit temperiertem Fluid möglich ist. Selbstverständlich können zu diesem Zweck auch separate Schlauchleitungen oder dergleichen vorgesehen sein.

Da das in bzw. durch den Strömungsbrecher 24 strömende Fluid nicht in Kontakt mit dem empfindlichen Inhalt des flexiblen Behälters 14 kommt, kann ein nicht steriles Fluid (z. B. Umgebungsluft oder normales Wasser) für die oben genannten Zwecke verwendet werden. Alternativ kann auch ein Filter vor das zugeführte Fluid geschaltet werden, um dieses steril in bzw. durch die Strömungsbrecher zu führen.

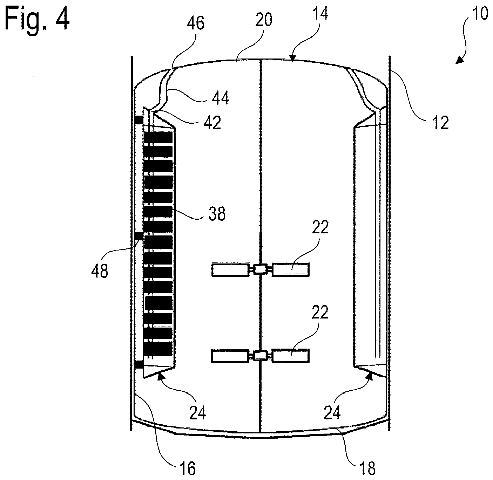

Eine weitere Ausführungsvariante ist in Figur 4 gezeigt. Hier sind mehrere Abstandshalter 48 zwischen der Seitenwand 16 des flexiblen Behälters 14 und einem Strömungsbrecher 24 angeordnet. Bei dieser Anordnung kann die Flüssigkeit im flexiblen Behälter 14 - vertikal betrachtet - zwischen den Abstandshaltern 48 durchströmen. Im Zusammenspiel mit den undurchdringbaren Strömungsbrechern 24 können weitere Verwirbelungen forciert und dadurch die Durchmischung begünstigt werden. Der mithilfe der Abstandshalter 48 erreichte radiale Abstand der Strömungsbrecher 24 von der Seitenwand 16 des flexiblen Behälters 14 liegt je nach Behältergröße in einem Bereich zwischen 0 und etwa 200 mm.

Abgesehen von den Strömungsbrechern 24 an der Seitenwand 16 des flexiblen Behälters 14 können grundsätzlich auch Strömungsbrecher an der Bodenwand 18 und/oder an der Deckenwand 20 angeordnet sein.

Wie eingangs bereits erwähnt ist die Erfindung nicht auf Einweg-Bioreaktoren beschränkt, sondern kann auch bei anderen beutelbasierten Mischvorrichtungen Anwendung finden.

### Bezuaszeichenliste

- 10: Mischvorrichtung
- 12: Stützbehälter
- 14: flexibler Behälter
- 16: Seitenwand
- 18: Bodenwand
- 20: Deckenwand
- 22: Rührorgan
- 24: Strömungsbrecher
- 26: Grundabschnitt
- 28: Lasche
- 30: Mantel
- 32: vertikale Abtrennung
- 34: Aufnahmeabschnitt
- 36: Aufnahmeabschnitt
- 38: Platte
- 40: horizontale Abtrennung
- 42: Fluidanschluss
- 44: Schlauchleitung
- 46: Fluidanschluss
- 48: Abstandshalter

## Patentansprüche

1. Mischvorrichtung (10) mit einem flexiblen Behälter (14), wenigstens einem Rührorgan (22) und wenigstens einem Strömungsbrecher (24),
wobei der Strömungsbrecher (24) auf der Innenseite an einer umlaufenden Seitenwand (16) des flexiblen Behälters (14) gebildet ist und ein in das Innere des Behälters (14) ragendes freies Ende aufweist,
**dadurch gekennzeichnet, dass** der Strömungsbrecher (24) zwei Laschen (28) zur Befestigung des Strömungsbrechers (24) an der umlaufenden Seitenwand (16) des flexiblen Behälters (14) aufweist.

2. Mischvorrichtung (10) nach Anspruch 1, **dadurch gekennzeichnet, dass** der Strömungsbrecher (24) einen Mantel (30) aufweist, der aus einem flexiblen Folienmaterial gebildet ist.

3. Mischvorrichtung (10) nach Anspruch 2, **dadurch gekennzeichnet, dass** der flexible Behälter (14) und der Mantel (30) aus dem gleichen Material gebildet sind.

4. Mischvorrichtung (10) nach Anspruch 2 oder 3, **dadurch gekennzeichnet, dass** der Mantel (30) einen doppellagigen Abschnitt aufweist.

5. Mischvorrichtung (10) nach einem der Ansprüche 2 bis 4, **dadurch gekennzeichnet, dass** der Mantel (30) durch eine vertikale Abtrennung (32) in zwei Aufnahmeabschnitte (34, 36) unterteilt ist, wobei die vertikale Abtrennung (32) als freies Ende des Strömungsbrechers (24) in das Innere des Behälters (14) ragt.

6. Mischvorrichtung (10) nach einem der Ansprüche 2 bis 5, **dadurch gekennzeichnet, dass** wenigstens eine formstabilisierende Platte (38) im Mantel (30) angeordnet ist.

7. Mischvorrichtung (10) nach einem der Ansprüche 2 bis 6, **dadurch gekennzeichnet, dass** der Mantel (30) mehrere abgetrennte Fächer aufweist, in denen jeweils eine Platte (38) aufgenommen ist.

8. Mischvorrichtung (10) nach einem der Ansprüche 2 bis 7, **dadurch gekennzeichnet, dass** der Mantel (30) oder der gesamte Strömungsbrecher (24) gasdicht abgeschlossen ist und wenigstens einen Fluidanschluss aufweist.

9. Mischvorrichtung (10) nach Anspruch 8, **dadurch gekennzeichnet, dass** der Mantel (30) oder der gesamte Strömungsbrecher (24) mit einem temperierten Fluid gefüllt sind.

10. Mischvorrichtung (10) nach einem der vorhergehenden Ansprüche, **dadurch gekennzeichnet, dass** die Laschen (28) durch Schweißen, Kleben oder eine vergleichbare Befestigungsmethode an der umlaufenden Seitenwand (16) befestigt sind.

11. Mischvorrichtung (10) nach einem der Ansprüche 2 bis 10, **dadurch gekennzeichnet, dass** sich die Laschen (28) auf entgegengesetzten Seiten eines dem Mantel (30) gegenüberliegenden Grundabschnitts (26) erstrecken.

12. Mischvorrichtung (10) nach einem der vorhergehenden Ansprüche, **dadurch gekennzeichnet, dass** der flexible Behälter (14) und die Laschen (28) aus dem gleichen Material gebildet sind.

13. Mischvorrichtung (10) nach einem der vorhergehenden Ansprüche, **dadurch gekennzeichnet, dass** zwischen der Innenseite des flexiblen Behälters (14) und dem Strömungsbrecher (24) mehrere Abstandshalter (48) angeordnet sind.

14. Mischvorrichtung (10) nach Anspruch 13, **dadurch gekennzeichnet, dass** die Abstandshalter (48) vertikal und/oder horizontal voneinander beabstandet sind.

15. Mischvorrichtung (10) nach einem der vorhergehenden Ansprüche, **dadurch gekennzeichnet, dass** der Strömungsbrecher (24) keine Durchgangsöffnungen aufweist.

## Claims

1. A mixing device (10) comprising a flexible container (14), at least one stirring element (22) and at least one baffle (24),
the baffle (24) being formed on the inside on a circumferential side wall (16) of the flexible container (14) and having a free end protruding into the interior of the container (14),
**characterized in that** the baffle (24) includes two tabs (28) for fixing the baffle (24) to the circumferential side wall (16) of the flexible container (14).

2. The mixing device (10) according to claim 1, **characterized in that** the baffle (24) has a shell (30) which is formed from a flexible film material.

3. The mixing device (10) according to claim 2, **characterized in that** the flexible container (14) and the shell (30) are formed from the same material.

4. The mixing device (10) according to claim 2 or 3, **characterized in that** the shell (30) includes a double-layered portion.

5. The mixing device (10) according to any of claims 2 to 4, **characterized in that** the shell (30) is divided into two receiving portions (34, 36) by means of a vertical partition (32), the vertical partition (32) protruding into the interior of the container (14) as a free end of the baffle (24).

6. The mixing device (10) according to any of claims 2 to 5, **characterized in that** at least one dimensionally stabilizing plate (38) is arranged in the shell (30).

7. The mixing device (10) according to any of claims 2 to 6, **characterized in that** the shell (30) includes several partitioned compartments, in each of which a plate (38) is accommodated.

8. The mixing device (10) according to any of claims 2 to 7, **characterized in that** the shell (30) or the entire baffle (24) is sealed in a gas-tight way and includes at least one fluid port.

9. The mixing device (10) according to claim 8, **characterized in that** the shell (30) or the entire baffle (24) is filled with a tempered fluid.

10. The mixing device (10) according to any of the preceding claims, **characterized in that** the tabs (28) are fixed to the circumferential side wall (16) by welding, gluing or a comparable fixing method.

11. The mixing device (10) according to any of claims 2 to 10, **characterized in that** the tabs (28) extend on opposite sides of a base portion (26) facing the shell (30).

12. The mixing device (10) according to any of the preceding claims, **characterized in that** the flexible container (14) and the tabs (28) are formed from the same material.

13. The mixing device (10) according to any of the preceding claims, **characterized in that** several spacers (48) are arranged between the inside of the flexible container (14) and the baffle (24).

14. The mixing device (10) according to claim 13, **characterized in that** the spacers (48) are spaced from each other vertically and/or horizontally.

15. The mixing device (10) according to any of the preceding claims, **characterized in that** the baffle (24) has no through openings.

## Revendications

1. Dispositif mélangeur (10), comportant un récipient flexible (14), au moins un organe d'agitation (22) et au moins une chicane (24),
la chicane (24) étant réalisée du côté intérieur sur une paroi latérale circonférentielle (16) du récipient flexible (14) et présentant une extrémité libre faisant saillie vers l'intérieur du récipient (14),
**caractérisé en ce que** la chicane (24) présente deux pattes (28) pour la fixation de la chicane (24) sur la paroi latérale circonférentielle (16) du récipient flexible (14).

2. Dispositif mélangeur (10) selon la revendication 1, **caractérisé en ce que** la chicane (24) présente une enveloppe (30) qui est réalisée en un matériau en feuille flexible.

3. Dispositif mélangeur (10) selon la revendication 2, **caractérisé en ce que** le récipient flexible (14) et l'enveloppe (30) sont réalisés en la même matière.

4. Dispositif mélangeur (10) selon la revendication 2 ou 3, **caractérisé en ce que** l'enveloppe (30) présente un tronçon à deux couches.

5. Dispositif mélangeur (10) selon l'une des revendications 2 à 4, **caractérisé en ce que** l'enveloppe (30) est divisée en deux tronçons de logement (34, 36) par une séparation verticale (32), la séparation verticale (32) faisant saille vers l'intérieur du récipient (14) en tant qu'extrémité libre de la chicane (24).

6. Dispositif mélangeur (10) selon l'une des revendications 2 à 5, **caractérisé en ce qu'**au moins une plaque (38) stabilisant la forme est agencée dans l'enveloppe (30).

7. Dispositif mélangeur (10) selon l'une des revendications 2 à 6, **caractérisé en ce que** l'enveloppe (30) présente plusieurs compartiments séparés dans lesquels une plaque (38) respective est logée.

8. Dispositif mélangeur (10) selon l'une des revendications 2 à 7, **caractérisé en ce que** l'enveloppe (30) ou l'entière chicane 24 est fermée de manière étanche au gaz et présente au moins un raccord de fluide.

9. Dispositif mélangeur (10) selon la revendication 8, **caractérisé en ce que** l'enveloppe (30) ou l'entière chicane (24) est remplie d'un fluide tempéré.

10. Dispositif mélangeur (10) selon l'une des revendications précédentes, **caractérisé en ce que** les pattes (28) sont fixées sur la paroi latérale circonférentielle (16) par soudage, par collage ou par une méthode de fixation comparable.

11. Dispositif mélangeur (10) selon l'une des revendications 2 à 10, **caractérisé en ce que** les pattes (28) s'étendent des côtés opposés d'un tronçon de base (26) faisant face à l'enveloppe (30).

12. Dispositif mélangeur (10) selon l'une des revendications précédentes, **caractérisé en ce que** le récipient flexible (14) et les pattes (28) sont réalisés en la même matière.

13. Dispositif mélangeur (10) selon l'une des revendications précédentes, **caractérisé en ce que** plusieurs écarteurs (48) sont agencés entre la face intérieure du récipient flexible (14) et la chicane (24).

14. Dispositif mélangeur (10) selon la revendication 13, **caractérisé en ce que** les écarteurs (48) sont espacés les uns des autres verticalement et/ou horizontalement.

15. Dispositif mélangeur (10) selon l'une des revendications précédentes, **caractérisé en ce que** la chicane (24) ne présente pas d'orifices de passage.
